# EUROPEAN PATENT SPECIFICATION

(11) **EP 4 082 576 B1**
(45) Date of publication and mention of the grant of the patent: **20.08.2025**
(21) Application number: 20907501.9
(22) Date of filing: 23.12.2020
(51) Int. Cl.: A61K 31/7088, A61K 45/00, A61K 39/395, A61P 1/16, A61P 3/00, C12Q 1/02, C12Q 1/34, A61K 31/712

(54) **BODY FAT REDUCING AGENT AND METHOD FOR SCREENING SUBSTANCE HAVING BODY FAT REDUCING EFFECT**
KÖRPERFETTREDUZIERMITTEL UND VERFAHREN ZUM SCREENEN EINER SUBSTANZ MIT KÖRPERFETTSENKENDER WIRKUNG
AGENT DE RÉDUCTION DE GRAISSE CORPORELLE ET PROCÉDÉ DE CRIBLAGE DE SUBSTANCE PRÉSENTANT UN EFFET DE RÉDUCTION DE GRAISSE CORPORELLE

(30) Priority: 24.12.2019 JP 2019232338; 19.05.2020 JP 2020087250
(43) Date of publication of application: 02.11.2022
(73) Proprietor: OSAKA UNIVERSITY, Suita-shi Osaka 565-0871 (JP); Innate Cell Therapy Inc., Osaka-shi, Osaka 530-0017 (JP)
(72) Inventor: AKIRA Shizuo, Suita-shi, Osaka 565-0871 (JP); CALDEZ MATIAS JOSE, Suita-shi, Osaka 565-0871 (JP); SAKAGUCHI Nobuo, Osaka-shi, Osaka 530-0017 (JP)
(74) Representative: Kraus & Lederer PartGmbB
(86) International application number: PCT/JP2020/048154
(87) International publication number: WO 2021/132329

(56) References cited:
- EP-A1- 3 769 784
- WO-A1-2019/182055
- CANSBY EMMELIE ET AL: "Targeted Delivery of Stk25 Antisense Oligonucleotides to Hepatocytes Protects Mice Against Nonalcoholic Fatty Liver Disease", CMGH CELLULAR AND MOLECULAR GASTROENTEROLOGY AND HEPATOLOGY, vol. 7, no. 3, 1 January 2019 (2019-01-01), pages 597 - 618, XP055892655, ISSN: 2352-345X, DOI: 10.1016/j.jcmgh.2018.12.004
- HABACHER CORNELIA; GUO YANWU; VENZ RICHARD; KUMARI POOJA; NEAGU ANCA; GAIDATZIS DIMOS; HARVALD EVA B; FÆRGEMAN NILS J; GUT HEINZ; : "Ribonuclease-Mediated Control of Body Fat", DEVELOPMENTAL CELL, vol. 39, no. 3, 13 October 2016 (2016-10-13), pages 359 - 369, XP029800823, DOI: 10.1016/j.devcel.2016.09.018
- N. PYDYN, J. KADLUCZKA, P. MAJOR, E. KUS, S. CHLOPICKI, A. BUDZYNSKI, J. JURA, J. KOTLINOWSKI: "P-01-007 Analysis of MCPIP1 level in NAFLD patients", FEBS OPEN BIO, vol. 9, no. Suppl. 1, 30 June 2019 (2019-06-30), US , pages 69, XP009537665, ISSN: 2211-5463

## Description

### TECHNICAL FIELD

The present invention relates to a body fat reducing agent and a method for screening for a substance capable of reducing body fat.

### BACKGROUND ART

Metabolic syndrome, which is a cluster of conditions including obesity and diabetes mellitus, is increasing with changes in diet. Metabolic syndrome is manifested in the liver as nonalcoholic fatty liver disease (NAFLD), which is also increasing. Fatty liver disease including NAFLD is a condition caused by triglyceride accumulation in the liver, and NAFLD is fatty liver disease that is not secondary to heavy alcohol consumption. NAFLD includes simple steatosis and nonalcoholic steatohepatitis (NASH). Simple steatosis is a benign condition that does not advance to a more severe form, whereas NASH may advance to liver cirrhosis or liver cancer. Common causes of NAFLD include obesity (visceral fat accumulation), diabetes mellitus, dyslipidemia, hypertension, rapid weight loss and acute fasting.

Effective therapy for viral hepatitis has recently been developed, but NAFLD and NASH have no effective therapy. Due to rapid increase in the number of patients, the development of curative therapy is desired.

Regnase-1, also known as ZC3H12A and MCPIP1, is a CCCH-type zinc finger protein. Regnase-1 harbors a ribonuclease domain in its central region and negatively regulates the immune response through Toll-like receptors (TLRs) and IL-1 receptors by degradation of target mRNAs, such as IL-6 and the p40 subunit of IL-12 (non-patent literatures 1 to 4). Regnase-1 deficiency in mice causes severe autoimmune disease, leading to hyperactivation of T cells. In nonimmune cells, Regnase-1 maintains iron homeostasis in the duodenal epithelium (non-patent literature 5). Mice deficient in Regnase-1 specifically in the intestinal epithelium cells are resistant to experimental colitis (non-patent literature 6). The role of Regnase-1 in the liver, however, has not been characterized.

### CITATION LIST

### NON-PATENT LITERATURE

Non-patent literature 1: Matsushita K, et al. (2009) Zc3h12a is an RNase essential for controlling immune responses by regulating mRNA decay. Nature 458(7242):1185-1190.
Non-patent literature 2: Iwasaki H, et al. (2011) The IkappaB kinase complex regulates the stability of cytokine-encoding mRNA induced by TLR-IL-1R by controlling degradation of regnase-1. Nat Immunol 12(12):1167-1175.
Non-patent literature 3: Uehata T, et al. (2013) Malt1-induced cleavage of regnase-1 in CD4(+) helper T cells regulates immune activation. Cell 153(5):1036-1049.
Non-patent literature 4: Mino T, et al. (2015) Regnase-1 and Roquin Regulate a Common Element in Inflammatory mRNAs by Spatiotemporally Distinct Mechanisms. Cell 161(5):1058-1073.
Non-patent literature 5: Yoshinaga M, et al. (2017) Regnase-1 Maintains Iron Homeostasis via the Degradation of Transferrin Receptor 1 and Prolyl-Hydroxylase-Domain-Containing Protein 3 mRNAs. Cell Rep 19(8):1614-1630.
Non-patent literature 6: Nagayama Y, et al. (2018) Regnase-1 controls colon epithelial regeneration via regulation of mTOR and purine metabolism. Proc Natl Acad Sci USA. 115(43):11036-11041.

### SUMMARY OF INVENTION

### TECHNICAL PROBLEM

An object of the invention is to examine the role of Regnase-1 in the liver and to provide a novel application of a Regnase-1 inhibitor.

### SOLUTION TO PROBLEM

The present invention was made to solve the above problems and includes the following.
(1) A body fat reducing agent comprising a Regnase-1 inhibitor as an active ingredient.
(2) The body fat reducing agent according to the above (1), wherein the agent is for improving metabolic syndrome.
(3) The body fat reducing agent according to the above (1), wherein the agent is for preventing and/or treating fatty liver disease.
(4) The body fat reducing agent according to the above (3), wherein the fatty liver disease is nonalcoholic fatty liver disease or nonalcoholic steatohepatitis.
(5) The body fat reducing agent according to any one of the above (1) to (4), wherein the Regnase-1 inhibitor is a nucleic acid capable of inhibiting the expression of Regnase-1.
(6) The body fat reducing agent according to any one of the above (1) to (4), wherein the Regnase-1 inhibitor is a gene therapy drug targeting a Regnase-1 gene.
(7) The body fat reducing agent according to any one of the above (1) to (4), wherein the Regnase-1 inhibitor is an antibody or peptide capable of specifically binding to Regnase-1.
(8) A method for screening for a substance capable of reducing body fat, the method comprising selecting a substance capable of inhibiting the expression of Regnase-1 or a substance capable of inhibiting the function of Regnase-1.
(9) The method according to the above (8), wherein the method comprises
   contacting a test substance with cells expressing Regnase-1,
   measuring the expression level of Regnase-1 in the cells, and
   comparing the expression level of Regnase-1 in the cells in contact with the test substance to that in the absence of the test substance to identify a substance capable of reducing the expression level of Regnase-1.
(10) The method according to the above (8), wherein the method comprises
   contacting a test substance with Regnase-1 and an RNA substrate,
   measuring the degradation level of the RNA substrate, and
   comparing the degradation level of the RNA substrate in contact with Regnase-1 in the presence of the test substance to that in the absence of the test substance to identify a substance capable of reducing the degradation level of the RNA substrate.

### ADVANTAGEOUS EFFECTS OF INVENTION

The present invention provides a body fat reducing agent comprising a Regnase-1 inhibitor as an active ingredient. The body fat reducing agent of the present invention is useful for improving metabolic syndrome, and for preventing and/or treating fatty liver disease. The present invention also provides a method for screening for a substance capable of reducing body fat. The screening method of the present invention can be used to identify a useful substance capable of reducing body fat.

### BRIEF DESCRIPTION OF DRAWINGS

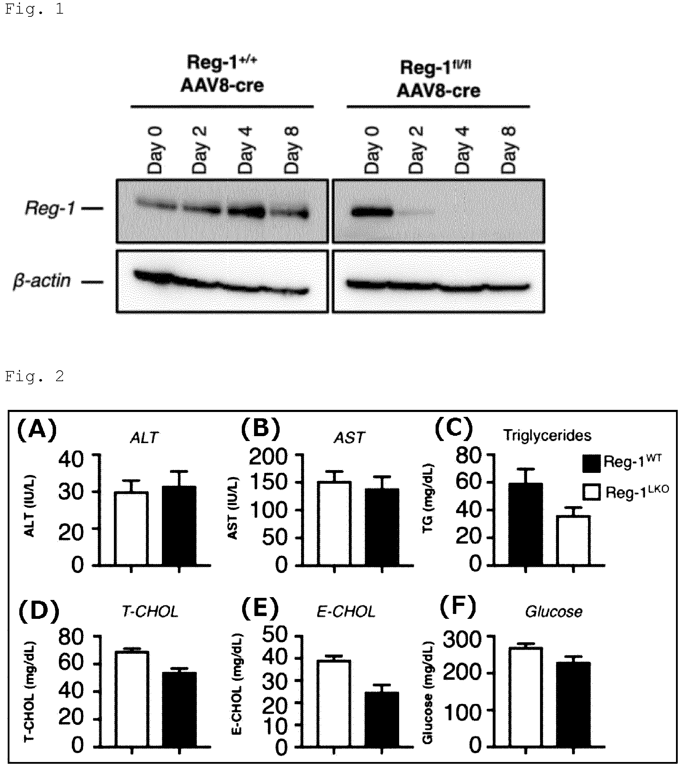

Fig. 1 shows Western blotting analysis of Regnase-1 in the liver of wild-type mice and Regnase-1^{fl/fl} mice carrying exons 4 to 6 of the Regnase-1 gene flanked by loxP sequences (Reg-1 flox/flox) on days 0, 2, 4 and 8 after administration of AAV.TBG.Cre (AddGene, #107787).
Fig. 2 shows charts showing the measurement results of (A) alanine transaminase (ALT), (B) aspartate aminotransferase (AST), (C) triglyceride, (D) total cholesterol, (E) esterified cholesterol and (F) glucose levels in the serum of liver-specific Regnase-1-deficient mice (Reg-1 LKO mice) and control mice (Reg-1 WT mice) three weeks after virus administration (at 8 weeks old).

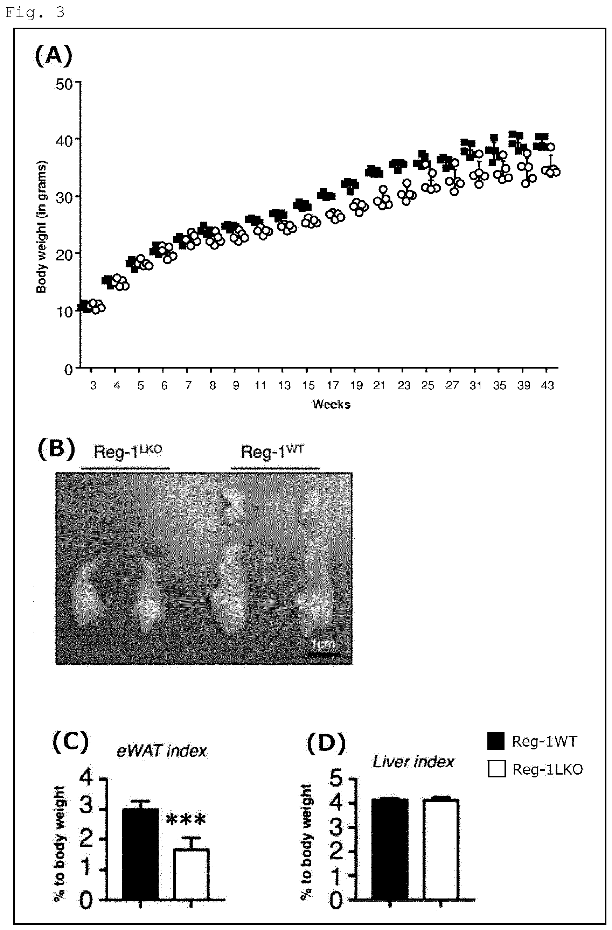

Fig. 3(A) shows a chart showing changes in the body weight of liver-specific Regnase-1 deficient mice (Reg-1 LKO mice) and control mice (Reg-1 WT mice) at the age of 3 to 43 weeks.
Fig. 3(B) shows a representative image of the epididymal white adipose tissue of Reg-1 LKO mice and Reg-1 WT mice at the age of 16 to 18 weeks.
Fig. 3(C) shows the epididymal white adipose tissue/body weight ratio (eWAT index) of Reg-1 LKO mice and Reg-1 WT mice at the age of 16 to 18 weeks.
Fig. 3(D) shows the liver to body weight ratio (Liver index) of Reg-1 LKO mice and Reg-1 WT mice at the age of 16 to 18 weeks.

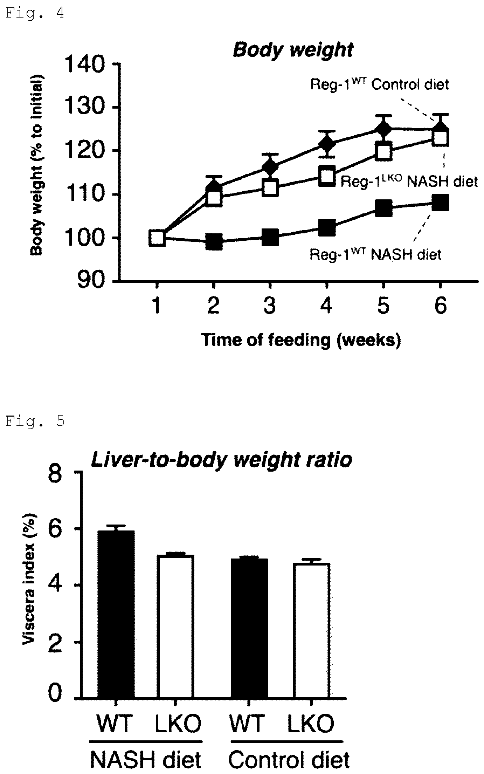

Fig. 4 shows a chart showing changes in the body weight of liver-specific Regnase-1 deficient mice (Reg-1 LKO mice) and control mice (Reg-1 WT mice) fed a NASH diet or a control diet for six weeks from week 2 after virus administration.
Fig. 5 shows a chart showing the liver to body weight ratio of mice in different groups six weeks after the start of feeding a NASH diet.

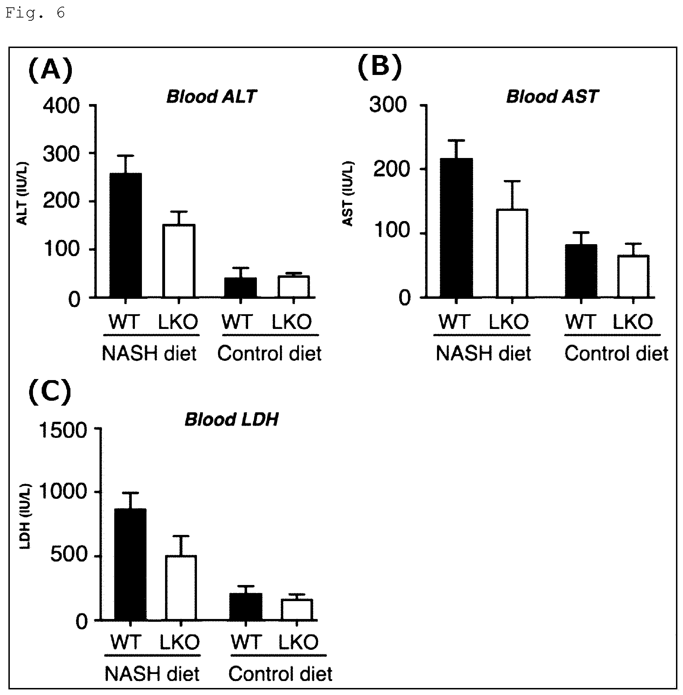

Fig. 6 shows charts showing (A) alanine transaminase (ALT), (B) aspartate aminotransferase (AST), and (C) lactate dehydrogenase (LDH) levels in the serum of mice in different groups six weeks after the start of feeding a NASH diet.

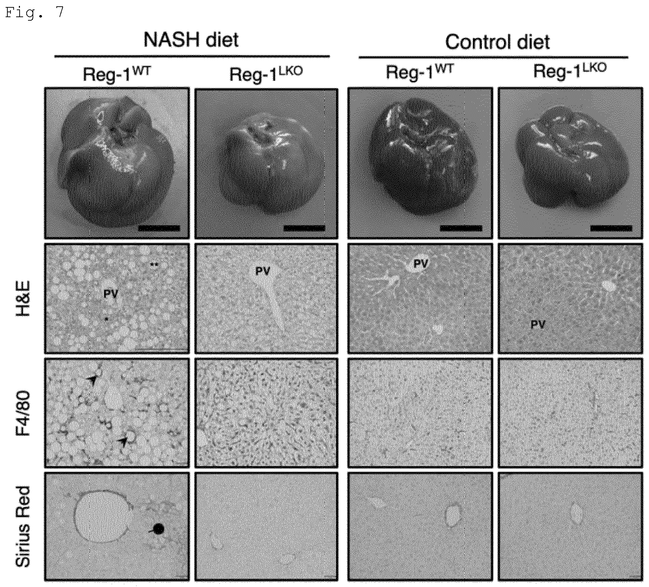

Fig. 7 shows images of histological analysis of the liver harvested from mice in different groups six weeks after the start of feeding a NASH diet. The images of the first row show the gross morphology of the liver. The images of the second row show hematoxylin and eosin staining. The images of the third row show immunostaining with anti-F4/80 antibody. The images of the fourth row show Sirius red staining.

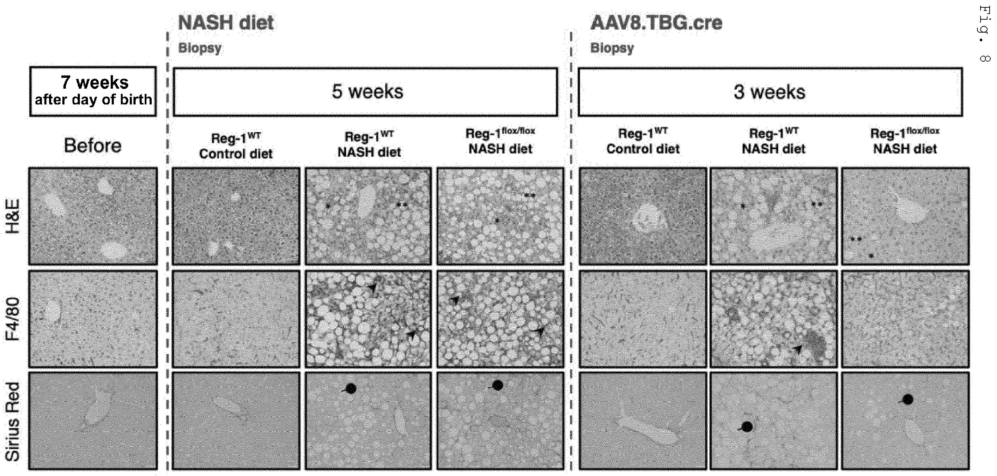

Fig. 8 shows images of histological analysis of the liver harvested from mice in different groups at different time points in experiments in which Regnase-1^{fl/fl} mice without liver-specific Regnase-1 deficiency were fed a NASH diet, and after manifestation of the pathological conditions of NASH, the mice received AAV.TBG.Cre to induce liver-specific Regnase-1 deficiency. The images of the first row show hematoxylin and eosin staining. The images of the second row show immunostaining with anti-F4/80 antibody. The images of the third row show Sirius red staining.

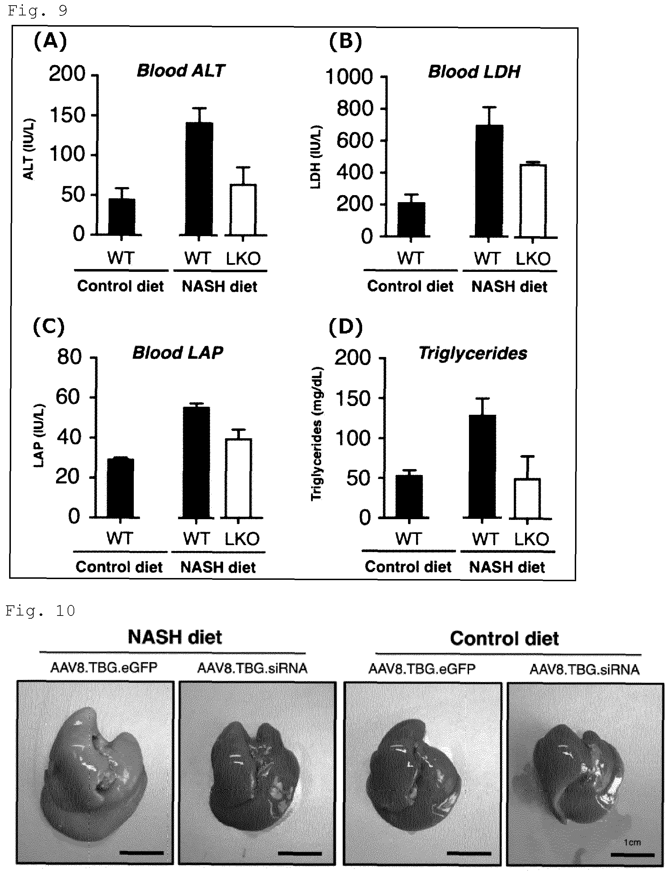

Fig. 9 shows charts showing (A) alanine transaminase (ALT), (B) lactate dehydrogenase (LDH), (C) leucine aminopeptidase (LAP), and (D) triglyceride levels in the serum of mice in different groups at week 3 after the administration of AAV.TBG.Cre in experiments in which Regnase-1^{fl/fl} mice without liver-specific Regnase-1 deficiency were fed a NASH diet, and after manifestation of the pathological conditions of NASH, the mice received AAV.TBG.Cre to induce liver-specific Regnase-1 deficiency.
Fig. 10 shows images showing the gross morphology of the liver harvested from mice in different groups at week 3 after viral infection in experiments in which C57/Bl6J mice were fed a NASH diet or a control diet for six weeks, and infected with AAV expressing an siRNA against the Regnase-1 gene or a control virus.

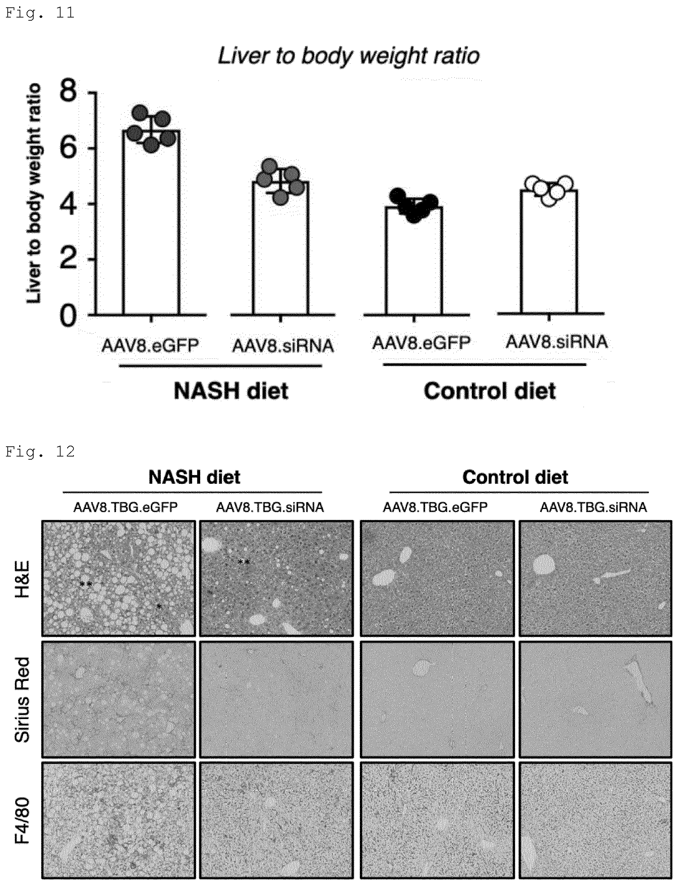

Fig. 11 shows a chart showing the liver to body weight ratio of mice in different groups calculated from the weight of the liver harvested from the mice at week 3 after viral infection in experiments in which C57/Bl6J mice were fed a NASH diet or a control diet for six weeks, and infected with AAV expressing an siRNA against the Regnase-1 gene or a control virus.
Fig. 12 shows images of histological analysis of the liver harvested from mice in different groups at week 3 after viral infection in experiments in which C57/Bl6J mice were fed a NASH diet or a control diet for six weeks, and infected with AAV expressing an siRNA against the Regnase-1 gene or a control virus. The images of the first row show hematoxylin and eosin staining. The images of the second row show Sirius red staining. The images of the third row show immunostaining with anti-F4/80 antibody.

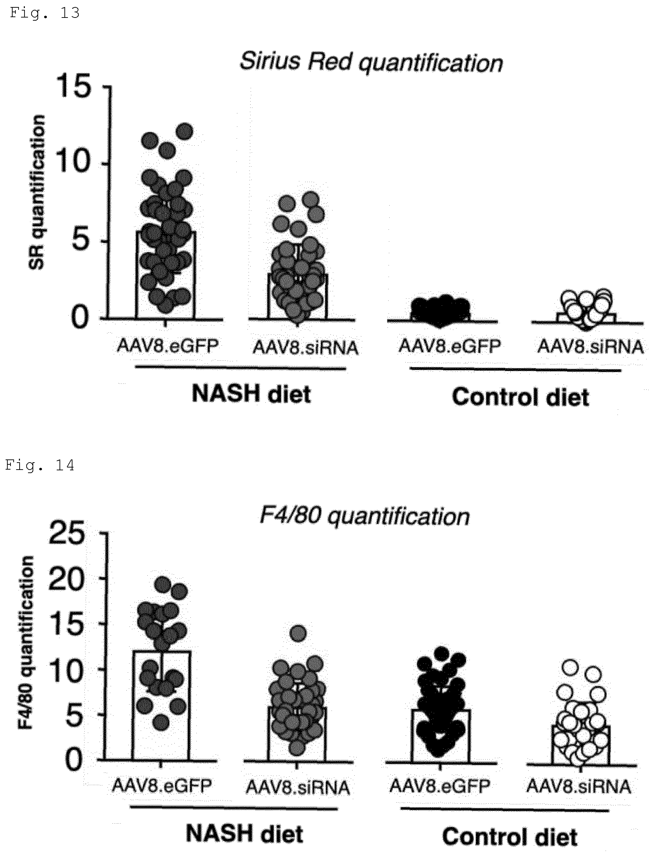

Fig. 13 shows a chart showing image analysis quantification of the positive area in the Sirius red stained specimens prepared from the liver harvested from mice in different groups at week 3 after viral infection in experiments in which C57/Bl6J mice were fed a NASH diet or a control diet for six weeks, and infected with AAV expressing an siRNA against the Regnase-1 gene or a control virus.
Fig. 14 shows a chart showing image analysis quantification of the anti-F4/80 antibody-positive area in the anti-F4/80 antibody immunostained specimens prepared from the liver harvested from mice in different groups at week 3 after viral infection in experiments in which C57/Bl6J mice were fed a NASH diet or a control diet for six weeks, and infected with AAV expressing an siRNA against the Regnase-1 gene or a control virus.

### DESCRIPTION OF EMBODIMENTS

### Body fat reducing agent

The present invention provides a body fat reducing agent comprising a Regnase-1 inhibitor as an active ingredient. The body fat reducing agent of the present invention is suitable for improving metabolic syndrome. The body fat reducing agent of the present invention is also suitable for preventing and/or treating fatty liver disease. Fatty liver disease is a condition caused by triglyceride accumulation in the liver, and is also called fatty liver. The fatty liver disease as described herein may be alcoholic fatty liver disease or nonalcoholic fatty liver disease (NAFLD). Ten to twenty percent of NAFLD cases may develop nonalcoholic steatohepatitis (NASH), which may lead to liver cirrhosis or liver cancer. The body fat reducing agent of the present invention can be used to prevent and/or treat NASH and is thus very useful.

The body fat reducing agent of the present invention can be formulated as a medicament. When formulated as a medicament, the body fat reducing agent can be formulated into a pharmaceutical formulation comprising a Regnase-1 inhibitor as an active ingredient by a conventional method. The body fat reducing agent may be formulated into a pharmaceutical formulation for oral administration in the form of, for example, solid and liquid dosage forms, and specific examples thereof include tablets (including sugar-coated tablets and film-coated tablets), pills, granules, powders, capsules (including soft capsules), syrups, emulsions, and suspensions. Such pharmaceutical formulations are prepared by a known method, and may contain a carrier, a diluent, or an excipient commonly used in the pharmaceutical field. For example, lactose, starch, sucrose, magnesium stearate, and the like are used as a carrier or an excipient for tablets. The body fat reducing agent may also be formulated into a pharmaceutical formulation for parenteral administration in the form of, for example, injections and suppositories. Examples of the injections may include injectable dosage forms, such as intravenous injections, subcutaneous injections, intradermal injections, intramuscular injections, intravenous drips, and intraarticular injections. Such injections are prepared by, for example, dissolving, suspending or emulsifying the active ingredient in a commonly used aseptic aqueous or oily solution for injection by a known method. The aqueous solution for injection may be, for example, physiological saline or an isotonic solution containing glucose or other auxiliary agents. The aqueous solution for injection may also contain an appropriate solubilizing agent, including, for example, alcohols (e.g., ethanol etc.), polyalcohols (e.g., propylene glycol, polyethylene glycol, etc.), and nonionic surfactants (e.g., polysorbate 80, HCO-50, etc.). The oily solution for injection may be, for example, sesame oil, soybean oil, or the like, and may also contain a solubilizing agent such as benzyl benzoate and benzyl alcohol. The body fat reducing agent may also be formulated into a suppository for rectal administration. The suppository is prepared by mixing the active ingredient with a commonly used base for suppositories. The pharmaceutical formulations prepared in these manners are safe and low toxic, and are safely administered orally or parenterally to humans or other mammals (e.g., rats, mice, rabbits, sheep, pigs, cows, cats, dogs, monkeys, etc.).

The body fat reducing agent of the present invention can be formulated as a food or drink. Such a food or drink includes health foods, foods with function claims, foods for special health use, foods for sick people, fortified foods, and dietary supplements. The form of the food or drink is not particularly limited. Specific examples of the food or drink include tablets, granules, powders, or energy drinks; drinks such as tea drinks, refreshing drinks, carbonated drinks, nutritional drinks, fruit juice, and lactic drinks; sweets and bakery products, such as drops, candies, chewing gum, chocolate, snacks, biscuits, jellies, jam, cream, pastries, and bread; fishery and livestock products, such as fish sausages, ham, and sausages; dairy products such as processed milk and fermented milk; fats, oils and processed foods thereof, such as vegetable oil, oil for deep frying, margarine, mayonnaise, shortening, whipped cream, and dressing; seasonings such as sauce and dipping sauce; retort pouch foods such as curry, stew, rice-bowl cuisine, porridge, and rice soup; and frozen desserts such as ice cream, sherbet, and shaved ice.

The active ingredient of the body fat reducing agent of the present invention may be a substance capable of inhibiting the expression of Regnase-1 or a substance capable of inhibiting the function of Regnase-1. The substance capable of inhibiting the expression of Regnase-1 may be a nucleic acid capable of inhibiting the expression of Regnase-1. The nucleic acid capable of inhibiting the expression of Regnase-1 may be a short interfering RNA (siRNA) against the Regnase-1 gene, a short hairpin RNA (shRNA) against the Regnase-1 gene, or an antisense oligonucleotide against the Regnase-1 gene. The nucleotide sequence of the Regnase-1 gene of an animal subjected to administration of the body fat reducing agent is easily available from known databases (e.g., DDBJ, GenBank, EMBL, etc.). The nucleic acid capable of inhibiting the expression of Regnase-1 can be designed using a known method. The accession Nos. of the nucleotide and amino acid sequences of the Regnase-1 gene of humans, mice and rats are, for example, as follows.
**Human: NM_001323550 (nucleotide sequence), NP_001310479 (amino acid sequence)**
   **NM_001323551 (nucleotide sequence), NP_001310480 (amino acid sequence)**
**Mouse: NM_153159 (nucleotide sequence), NP_694799 (amino acid sequence)**
**Rat: NM_001077671 (nucleotide sequence), NP_001071139 (amino acid sequence)**

siRNA is a double-stranded RNA of about 20 base pairs or less (e.g., about 21 to 23 base pairs) in length. When expressed in cells, siRNA inhibits the expression of a target gene (the Regnase-1 gene in the present invention). shRNA is a single-stranded RNA molecule of about 20 base pairs or more containing palindromic nucleotide sequences folded in an intramolecular double-stranded short hairpin structure with a 3' overhang. When introduced into cells, shRNA is degraded into a molecule of about 20 nucleotides in length (typically, e.g., 21, 22 or 23 nucleotides in length) and inhibits the expression of a target gene (the Regnase-1 gene in the present invention) in the same manner as in siRNA. The siRNA and shRNA of the present invention may be in any form that allows them to inhibit the expression of Regnase-1. The siRNA and shRNA can be designed by a known method based on the nucleotide sequence of a target gene. The siRNA and shRNA may be an artificial molecule that is chemically synthesized. Antisense or sense RNA can be synthesized in vitro from a template DNA using, for example, T7 RNA polymerase and T7 promoter. The antisense oligonucleotide of the present invention may be a nucleotide sequence that is complementary to or hybridizes to 5 to 100 contiguous nucleotides in the DNA sequence of the Regnase-1 gene, and may be a DNA or RNA. The antisense oligonucleotide may be modified to the extent that such a modification has no influence on the function of the antisense oligonucleotide. The antisense oligonucleotide can be synthesized by a conventional method. For example, the antisense oligonucleotide can be easily synthesized on a commercially available DNA synthesizer.

The nucleic acid capable of inhibiting the expression of Regnase-1 serving as an active ingredient of the body fat reducing agent of the present invention can be administered in the form of a non-viral vector or a viral vector. When administered in the form of a non-viral vector, the nucleic acid molecule can be administered into cells by liposomes (liposomes, HVJ-liposomes, cationic liposomes, lipofection, or lipofectamine), microinjection, injection with a carrier (metal particles) using a gene gun, or other methods. The nucleic acid may be administered in the form of siRNA or shRNA to a living body using a viral vector, such as a recombinant adenovirus or retrovirus. Specifically, a DNA that expresses the siRNA or shRNA is introduced into a DNA or RNA virus, such as attenuated retrovirus, adenovirus, adeno-associated virus, herpesvirus, vaccinia virus, poxvirus, poliovirus, Sindbis virus, Sendai virus or SV40, and the recombinant virus is used to infect cells or tissue to introduce the gene into the cells or tissue.

The sense strand of the siRNA is preferably identical to the target sequence, but is not necessarily completely identical to the target sequence as long as it can induce RNA interference. In particular, the sense strand of the siRNA may contain one or several (e.g., 2, 3 or 4) mismatches as long as its antisense strand hybridizes to the target sequence. In other words, the siRNA may have a sense strand containing one or several nucleotide substitutions, additions or deletions as compared to the target sequence and is capable of inducing RNA interference. The siRNA may have a sense strand that has 85% or more, 90% or more, 95% or more, or 98% or more sequence identity to the target sequence and is capable of inducing RNA interference.

The siRNA may be a hybrid siRNA in which all the nucleotides of the sense or antisense strand are replaced with DNA nucleotides, or a chimeric siRNA in which some of the nucleotides of the sense and/or antisense strand are replaced with DNA nucleotides as long as the siRNA is capable of inducing RNA interference. The hybrid siRNA may have a sense strand whose nucleotides are replaced with DNA nucleotides. The chimeric siRNA may be an siRNA in which some of the nucleotides in the downstream region (the 3' region of the sense strand or the 5' region of the antisense strand) are replaced with DNA nucleotides. Specifically, the nucleotides in the 3' region of the sense strand and the 5' region of the antisense strand may be replaced with DNA nucleotides, or some of the nucleotides in the 3' region of the sense strand or the 5' region of the antisense strand may be replaced with DNA nucleotides. The number of nucleotides replaced may be up to the number corresponding to half the length of the RNA molecule.

The siRNA may be a nucleotide analog having a chemically modified sugar, base and/or phosphate moiety in its nucleotides (ribonucleotides or deoxyribonucleotides). Examples of the nucleotide analog with a modified base include nucleotides having a 5-modified uridine or cytidine (e.g., 5-propynyluridine, 5-propynylcytidine, 5-methylcytidine, 5-methyluridine, 5-(2-amino)propyluridine, 5-halocytidine, 5-halouridine, 5-methyloxyuridine, etc.); nucleotides having a 8-modified adenosine or guanosine (e.g., 8-bromoguanosine etc.); deazanucleotides (e.g., 7-deaza-adenosine etc.); and O-alkylated and N-alkylated nucleotides (e.g., N⁶-methyladenosine etc.). Examples of the nucleotide analog with a modified sugar moiety include nucleotides having a 2'-modified sugar moiety in which 2'-OH of the ribonucleotide is substituted with H, OR, R, a halogen atom, SH, SR, NH₂, NHR, NR₂ or CN (wherein R represents an alkyl, alkenyl or alkynyl group of 1 to 6 carbon atoms), and 5'-phosphorylated nucleotides in which the 5' end is monophosphorylated. Examples of the nucleotide analog with a modified phosphate moiety include nucleotides in which the phosphodiester groups that form a linkage between the adjacent ribonucleotides are substituted with phosphorothioate groups.

The substance capable of inhibiting the expression of Regnase-1 may be a gene therapy drug targeting the Regnase-1 gene. Such a gene therapy drug may be, for example, a viral vector containing genome editing means targeting the Regnase-1 gene. The viral vector may be, for example, lentiviral vectors, adenoviral vectors, or adeno-associated viral vectors. The genome editing means may be CRISPER/Cas9. CRISPER/Cas9 is composed of Cas9 protein and guide RNA, and the guide RNA may contain a nucleotide region complementary to the Regnase-1 gene and a region interacting with the Cas9 protein. The gene therapy drug is preferably an adeno-associated viral vector containing a polynucleotide that expresses CRISPER/Cas9 targeting the Regnase-1 gene operably linked to a liver-specific promoter. Such a viral vector containing genome editing means targeting the Regnase-1 gene is used to infect cells or tissue to disrupt or replace the Regnase-1 gene and inhibit the expression of Regnase-1.

The active ingredient of the body fat reducing agent of the present invention may be an antibody or peptide capable of specifically binding to Regnase-1. The antibody or peptide binds to Regnase-1 to inhibit the binding of Regnase-1 to an RNA substrate. The antibody capable of specifically binding to Regnase-1 may be a polyclonal antibody or a monoclonal antibody. The antibody may be a full-length antibody molecule, or an antibody fragment capable of specifically binding to Regnase-1 (e.g., Fab, F(Ab')₂, Fab', Fv, scFv, etc.). The antibody may be a human chimeric antibody or a humanized antibody. The antibody and peptide may be produced by a known method. Such a peptide or antibody serving as the active ingredient of the medicament of the present invention is preferably combined with a pharmaceutically acceptable carrier and formulated into an injection or an infusion, and administered via a parenteral route, for example, an intravenous, intramuscular, intradermal, intraperitoneal, subcutaneous or local route of administration.

The active ingredient of the body fat reducing agent of the present invention may be a low-molecular-weight compound capable of inhibiting the expression or function of Regnase-1.

The amount of the active ingredient contained in the body fat reducing agent of the present invention may be 0.001 to 50% by mass, and is preferably 0.01 to 10% by mass, more preferably 0.1 to 1% by mass. The dosage of the medicament of the present invention is determined as appropriate depending on the purpose of use, the type of disease, the severity of the disease, the age, body weight and sex of the patient, the past medical history, the type of active ingredient, or other factors. The dosage is preferably about 0.02 mg to 4000 mg, more preferably about 0.1 mg to 200 mg, per day for an average human with a body weight of about 65 to 70 kg. The total daily dosage may be administered in a single dose or several divided doses.

### Screening method

The present invention provides a method for screening for a substance capable of reducing body fat. The screening method of the present invention uses Regnase-1. Regnase-1 used in the screening method of the present invention may be Regnase-1 protein or the Regnase-1 gene. Regnase-1 protein may be a full-length protein or an active fragment thereof containing a ribonuclease domain.

Regnase-1 used in the screening method of the present invention may be from any organism. Regnase-1 used in the screening method of the present invention may be from a mammal. The mammal may be, for example, a human, a chimpanzee, a monkey, a dog, a cow, a mouse, a rat, or a guinea pig, and is preferably a human. Information on the nucleotide and amino acid sequences of the gene encoding Regnase-1 of various animals is available from known databases (DDBJ, GenBank, EMBL, etc.). The accession Nos. of the nucleotide and amino acid sequences of the Regnase-1 gene of humans, mice and rats are, for example, as follows.
**Human:** NM_001323550 (nucleotide sequence), NP_001310479 (amino acid sequence)
   **NM_001323551 (nucleotide sequence), NP_001310480 (amino acid sequence)**
**Mouse: NM_153159 (nucleotide sequence),** NP_694799 (amino acid sequence)
**Rat: NM_001077671 (nucleotide sequence), NP_001071139 (amino acid sequence)**

A test substance to be subjected to the screening method of the present invention may be, for example, but is not limited to, a nucleic acid, a peptide, a protein, a non-peptidic compound, a synthetic compound, a fermentation product, a cell extract, a cell culture supernatant, a plant extract, a mammalian tissue extract, a plasma, or the like. The test substance may be a novel substance or a known substance. The test substance may be in the form of a salt. The salt of the test substance may be a salt with a physiologically acceptable acid or base.

The screening method of the present invention may be used to select a substance capable of inhibiting the expression of Regnase-1. The screening method of the present invention used to select a substance capable of inhibiting the expression of Regnase-1 may comprise, for example, contacting a test substance with cells expressing Regnase-1, measuring the expression level of Regnase-1 in the cells, and comparing the expression level of Regnase-1 in the cells in contact with the test substance to that in the absence of the test substance to identify a substance capable of reducing the expression level of Regnase-1.

The cells expressing Regnase-1 may be cells in a living body or cultured cells. The cultured cells may be primary cultured cells or an established cell line. The cells expressing Regnase-1 may be cells expressing endogenous Regnase-1, or cells expressing exogenous Regnase-1. The cells expressing endogenous Regnase-1 may be, for example, HeLa cells, HEK293 cells, Jurkat cells, Caco-2 cells, mouse fetal fibroblasts (MEF), or EL-4 cells. The cells expressing exogenous Regnase-1 can be produced by introducing a recombinant expression vector carrying DNA encoding Regnase-1 (Regnase-1 expression vector) into appropriate host cells. The host cells may be, for example, MEF cells or primary cultured cells from Regnase-1 deficient mice; or HeLa cells, HEK293 cells, Jurkat cells, Caco-2 cells, or EL-4 cells.

The test substance may be brought into contact with cells in any appropriate manner. For example, when cultured cells are used, the test substance may be allowed to contact with the cells by adding the test substance to the culture medium. For example, when the test substance is brought into contact with cells in a living body of a non-human animal, the test substance may be systemically administered to the non-human animal via oral, intravenous or intraperitoneal route, or locally administered to the target organ or tissue. Preferably, a control group in the absence of contact with the test substance is provided.

The expression level of Regnase-1 can be measured as the protein level of Regnase-1 or as the mRNA level of Regnase-1. The protein level of Regnase-1 can be measured by extracting the protein from cells by a known method and quantifying the protein by a known protein measurement method. Examples of the known protein measurement method include Western blotting, EIA, ELISA, RIA, and a method using a protein measurement reagent. The mRNA level of Regnase-1 can be measured by extracting RNA from cells by a known method and quantifying the mRNA by a known mRNA measurement method. Examples of the known mRNA measurement method include Northern blotting, RT-PCR, quantitative RT-PCR, and RNase protection assay.

The test substance can be identified as a substance of interest when the protein or mRNA level of Regnase-1 in cells made in contact with the test substance is reduced as compared to that in a control group in the absence of contact with the test substance. The degree of reduction in the protein or mRNA level of Regnase-1 by the test substance is not particularly limited, but, for example, the test substance may be identified as a substance of interest when it reduces the protein or mRNA level of Regnase-1 to 50% or less, 40% or less, 30% or less, 20% or less, or 10% or less of that in cells in the absence of contact with the test substance.

The screening method of the present invention may be used to select a substance capable of inhibiting the function of Regnase-1. The function of Regnase-1 may be endoribonuclease activity. The screening method of the present invention used to select a substance capable of inhibiting the endoribonuclease activity of Regnase-1 may comprise, for example, contacting a test substance with Regnase-1 and an RNA substrate, measuring the degradation level of the RNA substrate, and comparing the degradation level of the RNA substrate in contact with Regnase-1 in the presence of the test substance to that in the absence of the test substance to identify a substance capable of reducing the degradation level of the RNA substrate.

The method for screening for a substance capable of inhibiting the endoribonuclease activity of Regnase-1 may be performed in a system containing cells expressing Regnase-1 or in a system not containing cells expressing Regnase-1. When the screening method is performed in a system not containing cells expressing Regnase-1, Regnase-1 and a RNA substrate and a test substance are added to an appropriate reaction buffer (e.g., Tris buffer solution containing magnesium, e.g., 20 mM Tris-HCl (pH 7.5), 150 mM NaCl, 5 mM MgCl₂ and 1 mM DTT), then reacted at 30 to 37°C for 30 to 120 minutes, and the degradation (cleavage) level of the RNA substrate is measured. When the test substance inhibits the endoribonuclease activity of Regnase-1, the cleavage of the RNA substrate is inhibited and the degradation level is reduced.

Recombinant Regnase-1 protein is suitable for use in the system not containing cells expressing Regnase-1. The recombinant Regnase-1 protein may be a full-length protein or an active fragment thereof containing a ribonuclease domain. The RNA substrate may be a RNA containing a sequence known to be cleaved by Regnase-1. The RNA substrate may be a synthetic RNA.

The degradation level of the RNA substrate may be measured by any method including, for example, but not limited to, PCR or electrophoresis. For example, when the degradation level is measured by PCR, a chimeric oligo containing DNA sequences at both 5' and 3' ends of the RNA substrate may be synthesized and used as the RNA substrate. In this reaction system, primers complementary to the DNA sequences at the 5' and 3' ends of the chimeric oligo substrate are used to perform quantitative PCR. Cleavage of the chimeric oligo substrate results in reduction in the PCR amplification efficiency, whereas non-cleavage of the chimeric oligo substrate results in increase in the PCR amplification efficiency. In other words, if the test substance is a substance capable of inhibiting the endoribonuclease activity of Regnase-1, the PCR amplification efficiency is higher than that in the absence of the test substance (Regnase-1 cleaves the chimeric oligo substrate). Such a test substance can be identified as a substance capable of reducing the degradation level of the RNA substrate. In this reaction system, the test substance may be identified as a substance of interest when it increases the PCR amplification level to 1.5-fold or more, 1.6-fold or more, 1.7-fold or more, 1.8-fold or more, 1.9-fold or more, or 2-fold or more that in the absence of the test substance.

For example, when the degradation level is measured by electrophoresis, the above chimeric oligo substrate may be labeled at one or both ends and used as the RNA substrate. The labeling substance may be any substance that can be used to quantify the intensity of the bands of electrophoresis, and may be, for example, a fluorescent label, a RI label, or a biotin label. When the chimeric oligo substrate labeled with a fluorescence at both ends is used for reaction and collected and subjected to electrophoresis, the chimeric oligo substrate not cleaved by Regnase-1 appears as a single full-length band labeled with the fluorescence at both ends. The chimeric oligo substrate cleaved by Regnase-1 appears as at least two shorter bands or further degraded fragments. In other words, if the test substance is a substance capable of inhibiting the endoribonuclease activity of Regnase-1, a single full-length band is predominantly observed as compared to electrophoresis of the reaction in the absence of the test substance (Regnase-1 cleaves the chimeric oligo substrate). Such a test substance can be identified as a substance capable of reducing the degradation level of the RNA substrate.

When the method for screening for a substance capable of inhibiting the endoribonuclease activity of Regnase-1 is performed in a system containing cells expressing Regnase-1, the cells expressing Regnase-1 may be cells expressing endogenous Regnase-1, or cells expressing exogenous Regnase-1. The cells expressing exogenous Regnase-1 may be Regnase-1 expressing cells produced by introducing a Regnase-1 expression vector into host cells. The RNA substrate may be a RNA containing a sequence known to be cleaved by Regnase-1. For example, the RNA substrate may be a transcript product of the 3'-UTR of the IL-6 gene.

In this reaction system, the cells expressing Regnase-1 may be produced by introducing a Regnase-1 expression vector and a vector containing DNA encoding the 3'-UTR of the IL-6 gene linked downstream of a luciferase gene into appropriate host cells. After a test substance is added to the culture medium, the cells are cultured for about one to three days (preferably for two days). The cultured cells are then lysed, and the luciferase activity in the cell lysate is measured by a known method. When these cells are used, the 3'-UTR region of mRNA of the luciferase gene is cleaved by Regnase-1, resulting in reduction in the luciferase activity. If the endoribonuclease activity of Regnase-1 is inhibited, the 3'-UTR region of mRNA of the luciferase gene is not cleaved, resulting in maintenance of the luciferase activity.

In other words, if the test substance is a substance capable of inhibiting the endoribonuclease activity of Regnase-1, the luminescence intensity is higher than that in the reaction in the absence of the test substance (Regnase-1 cleaves the chimeric oligo substrate). Such a test substance can be identified as a substance capable of reducing the degradation level of the RNA substrate. In this reaction system, the test substance may be identified as a substance of interest when it increases the luminescence intensity to 1.5-fold or more, 1.6-fold or more, 1.7-fold or more, 1.8-fold or more, 1.9-fold or more, or 2-fold or more that in the absence of the test substance.

The present invention further includes the following.
(A1) A method for reducing body fat, the method comprising administering an effective amount of a substance capable of inhibiting the expression of Regnase-1 or a substance capable of inhibiting the function of Regnase-1.
(A2) A method for improving metabolic syndrome, the method comprising administering an effective amount of a substance capable of inhibiting the expression of Regnase-1 or a substance capable of inhibiting the function of Regnase-1.
(A3) A method for preventing and/or treating fatty liver disease, the method comprising administering an effective amount of a substance capable of inhibiting the expression of Regnase-1 or a substance capable of inhibiting the function of Regnase-1.
(B1) Use of a substance capable of inhibiting the expression of Regnase-1 or a substance capable of inhibiting the function of Regnase-1 for the reduction of body fat.
(B2) A substance capable of inhibiting the expression of Regnase-1 or a substance capable of inhibiting the function of Regnase-1 for use in the improvement of metabolic syndrome.
(B3) A substance capable of inhibiting the expression of Regnase-1 or a substance capable of inhibiting the function of Regnase-1 for use in the prevention and/or treatment of fatty liver disease.
(C1) Use of a substance capable of inhibiting the expression of Regnase-1 or a substance capable of inhibiting the function of Regnase-1 for the production of a body fat reducing agent.
(C2) Use of a substance capable of inhibiting the expression of Regnase-1 or a substance capable of inhibiting the function of Regnase-1 for the production of a metabolic syndrome improving agent.
(C3) Use of a substance capable of inhibiting the expression of Regnase-1 or a substance capable of inhibiting the function of Regnase-1 for the production of a medicament for preventing and/or treating fatty liver disease.

### EXAMPLES

The present invention will be described in more detail below with reference to Examples, but the present invention is not limited thereto.

All the following experiments were performed using mice with a C57/Bl6 background. Mice carrying exons 4 to 6 of the Regnase-1 gene flanked by loxP sequences (Reg-1 flox/flox) were generated (Regnase-1^{fl/fl} mice) as described in Uehata et al. (Uehata T, et al. (2013) Malt1-induced cleavage of regnase-1 in CD4(+) helper T cells regulates immune activation. Cell 153(5):1036-1049.).

### Example 1: Examination of liver of liver-specific Regnase-1-deficient mice

### (1) Generation of liver-specific Regnase-1-deficient mice

Regnase-1^{fl/fl} mice at 5 weeks old were received AAV.TBG.Cre (AddGene, #107787) at 0.625 × 10¹² vp/100 µL PBS via the tail vein to generate liver-specific Regnase-1-deficient mice (Reg-1 LKO mice). Control mice received AAV8 viral particles via the tail vein (Reg-1 WT mice). The Regnase-1 gene was completely deleted from Reg-1 LKO mice within two days (Fig. 1).

### (2) Biochemical analysis

The blood was collected from Reg-1 LKO mice and Reg-1 WT mice three weeks after virus administration (at 8 weeks old), and the serum was separated and analyzed by biochemical tests to examine the effects of the deletion of Regnase-1 in the liver. The results are shown in Fig. 2. Alanine transaminase (ALT) and aspartate aminotransferase (AST) levels in Reg-1 LKO mice were comparable to those of Reg-1 WT mice, indicating that no hepatic injury was induced by the deletion of Regnase-1. Triglyceride (Triglycerides), total cholesterol (T-CHOL), and esterified cholesterol (E-CHOL) levels in the serum of Reg-1 LKO mice were reduced as compared to those of Reg-1 WT mice, suggesting that Regnase-1 is involved in regulation of fat metabolism in the liver. The serum glucose level (Glucose) of Reg-1 LKO mice was also reduced as compared to that of Reg-1 WT mice, suggesting that Regnase-1 is also involved in sugar metabolism in the liver.

### (3) Changes in body weight

Body weight of Reg-1 LKO mice and Reg-1 WT mice was measured at the age of 3 to 43 weeks, and changes in the body weight are shown in Fig. 3(A). The body weight gain was lower in Reg-1 LKO mice than that in Reg-1 WT mice after week 9. The major cause of lower body weight gain was presumed reduction of body fat. Fig. 3(B) shows a representative image of the epididymal white adipose tissue of Reg-1 LKO mice and Reg-1 WT mice at the age of 16 to 18 weeks. Reg-1 LKO mice apparently had a small amount of adipose tissue. Fig. 3(C) shows the epididymal white adipose tissue/body weight ratio (eWAT index) of Reg-1 LKO mice and Reg-1 WT mice at the age of 16 to 18 weeks. Fig. 3(D) shows the liver to body weight ratio (Liver index) of Reg-1 LKO mice and Reg-1 WT mice at the age of 16 to 18 weeks. The eWAT index of Reg-1 LKO mice was significantly lower than that of Reg-1 WT mice, whereas the Liver index was comparable between Reg-1 LKO mice and Reg-1 WT mice, as shown in Figs. 3(C) and 3(D).

### (4) Summary

The results demonstrate that liver-specific Regnase-1 deficiency reduces body fat and the blood sugar levels without causing severe pathology, suggesting that inhibition of Regnase-1 in the liver may lead to improvement of metabolic syndrome.

### Example 2: Effect of NASH diet in liver-specific Regnase-1-deficient mice

Reg-1 LKO mice and Reg-1 WT mice were fed a NASH diet (Oriental Yeast Co., Ltd.; NASH-1) or a control diet from week 2 after virus administration. After feeding the NASH diet or the control diet for six weeks, samples were collected from the mice.

### (1) Changes in body weight

The body weight was measured every other week from the start of feeding the NASH diet. The results are shown in Fig. 4. Body weight gain was observed for the control diet group and the NASH diet group of Reg-1 WT mice, but the body weight gain of the NASH diet group was lower than that of the control diet group. The NASH diet group of Reg-1 LKO mice displayed body weight gain comparable to that of the control diet group of Reg-1 WT mice.

### (2) Liver weight

Liver was harvested from the mice six weeks after the start of feeding the NASH diet to measure the weight, and the liver to body weight ratio (a measure of liver inflammation) was calculated. The results are shown in Fig. 5. The liver/body weight ratio was higher in the NASH diet group of Reg-1 WT mice than that in the other groups (including the control diet group of Reg-1 WT mice and the NASH diet group and the control diet group of Reg-1 LKO mice). This results demonstrate that liver inflammation was induced by ingestion of the NASH diet in mice without liver-specific Regnase-1 deficiency (Reg-1 WT mice), whereas no liver inflammation was induced by ingestion of the NASH diet in Reg-1 LKO mice.

### (3) Biochemical analysis

The serum was collected from the mice in the different groups to measure alanine transaminase (ALT), aspartate aminotransferase (AST) and lactate dehydrogenase (LDH) levels. The results are shown in Fig. 6. The ALT, AST and LDH levels were high in the NASH diet group of Reg-1 WT mice, but were significantly reduced in the NASH diet group of Reg-1 LKO mice.

### (4) Histological analysis

Liver was harvested from the mice in the different groups for gross observation and histological analysis. Tissue specimens were prepared and stained with hematoxylin and eosin (H&E) staining, immunostaining with anti-F4/80 antibody, and Sirius red staining. The results are shown in Fig. 7. Steatosis around the portal vein (pv) (H&E staining), accumulation of macrophages (F4/80 positive cells) and fibrosis (Sirius red staining) were more severe in the NASH diet group of Reg-1 WT mice. The NASH diet group of Reg-1 LKO mice, however, showed significant reduction in steatosis around the portal vein, accumulation of macrophages and fibrosis.

### (5) Summary

The results demonstrate that liver-specific Regnase-1 deficiency in diet-induced NASH model mice induces reduction in hepatic inflammation and injury, suggesting that inhibition of Regnase-1 in the liver may prevent the onset of NASH.

### Example 3: Study of liver-specific Regnase-1 deficiency-mediated improvement of pathological conditions of NASH after manifestation of the pathological conditions of NASH

Regnase-1^{fl/fl} male mice at the age of 6 to 7 weeks were fed a NASH diet (Oriental Yeast Co., Ltd.; NASH-1) for five weeks. AAV.TBG.Cre was administered to the mice via the tail vein in the same manner as in Example 1. As positive and negative controls, Regnase-1^{fl/fl} mice at the age of 6 to 7 weeks were fed the NASH diet or a control diet for weeks, and AAV8 viral particles were administered in place of AAV.TBG.Cre. (the NASH diet group of Reg-1 WT mice and the control diet group of Reg-1 WT mice) (see Fig. 8).

### (1) Histological analysis

Liver was harvested from the mice in the different groups for histological analysis before the start of feeding the NASH diet, and at week 5 after the start of feeding the NASH diet, and at week 3 after the administration of AAV.TBG.Cre. Tissue specimens were prepared and stained with hematoxylin and eosin (H&E) staining, immunostaining with anti-F4/80 antibody, and Sirius red staining. The results are shown in Fig. 8. Mice fed the NASH diet for five weeks (the NASH diet groups of Reg-1 WT mice and Regnase-1^{fl/fl} mice) manifested the pathological conditions of NASH, including micro- and macrovesicular steatosis (H&E staining), inflammation (F4/80 positive cells), and fibrosis (Sirius red staining). After administration of AAV8 viral particles, the NASH diet group of Reg-1 WT mice was continuously fed the NASH diet for another three weeks, and showed exacerbation of micro- and macrovesicular steatosis. In contrast, the mice received AAV.TBG.Cre to induce liver-specific Regnase-1 deficiency showed significant reduction of micro- and macrovesicular steatosis, inflammation and fibrosis.

### (2) Biochemical analysis

The blood was collected from the mice at week 3 after the administration of AAV.TBG.Cre or AAV8 viral particles, and the serum was separated and used for biochemical tests. The results are shown in Fig. 9. The mice received AAV.TBG.Cre to induce liver-specific Regnase-1 deficiency (LKO in Fig. 9) showed significant reduction in the hepatic injury markers, ALT, LDH and LAP. The mice had almost no increase in the serum triglyceride level.

### (3) Summary

The results demonstrate that inhibition of Regnase-1 in the liver may improve inflammation, fibrosis and fat metabolism disorders, which serve as indicators of the pathological conditions of NASH. This indicates that Regnase-1 in the liver can be a promising target for treatment of fatty liver disease.

### Example 4: Study of improvement of pathological conditions of NASH by administration of adeno-associated virus (AAV) that liver-specifically expresses siRNA against Regnase-1 gene in NASH model mice

Adeno-associated virus (AAV) expressing an siRNA against the Regnase-1 gene was prepared using thyroxine-binding globulin (TBG) promoter, which is a liver specific promoter. The siRNA against the Regnase-1 gene had an identical nucleotide sequence to that of a commercially available siRNA targeting the mouse Regnase-1 gene (Thermo Fisher Scientific, siRNA ID: 170484, Catalog#: AM16708). As a control virus, AAV expressing GFP gene in place of the siRNA against the Regnase-1 gene was used.

C57/Bl6J mice at the age of 5 weeks were fed the NASH diet or a control diet for six weeks, and received the AAV expressing the siRNA against the Regnase-1 gene or the control virus (1.8 × 10¹² vp) via the tail vein for establishment of viral infection.

### (1) Gross observation and weight measurement of liver

Liver was harvested from the mice in the different groups three weeks after virus administration for gross observation and weight measurement. Fig. 10 shows the gross morphology of the liver, and Fig. 11 shows the liver to body weight ratio (a measure of liver inflammation). The NASH diet group mice infected with the control virus had characteristic yellowish fatty liver that was larger in size than that of the control diet group mice. In contrast, the liver of the NASH diet group mice infected with the AAV expressing the siRNA against the Regnase-1 gene was comparable in size and color to those of the control diet group mice.

### (2) Histological analysis

After the liver weight was measured, liver tissue specimens were prepared and stained with hematoxylin and eosin (H&E) staining, Sirius red staining, and immunostaining with anti-F4/80 antibody. The results are shown in Fig. 12. Fig. 13 shows image analysis quantification of the positive area (fibrosis) in the Sirius red stained specimens. Fig. 14 shows image analysis quantification of the positive area (inflammation) in the anti-F4/80 antibody immunostaining. The NASH diet group mice infected with the AAV expressing the siRNA against the Regnase-1 gene showed significant reduction of micro- and macrovesicular steatosis, which is a pathological condition of NASH, as compared to the NASH diet group mice infected with the control virus. The Sirius red staining and its quantification as shown in Fig. 13 showed that fibrosis was reduced in the NASH diet group mice infected with the AAV expressing the siRNA against the Regnase-1 gene. The quantification of the anti-F4/80 antibody immunostaining as shown in Fig. 14 showed that accumulation of macrophages (inflammation) was reduced.

### (3) Summary

The results suggest that gene therapy using an siRNA against the Regnase-1 gene may be a promising therapy for NASH.

The present invention is not limited to each of the embodiments and Examples as described above, and various modifications are possible within the scope of the claims.

## Claims

1. A Regnase-1 inhibitor for use in the prevention and/or treatment of fatty liver disease.

2. The Regnase-1 inhibitor for use according to claim 1, wherein the fatty liver disease is nonalcoholic fatty liver disease or nonalcoholic steatohepatitis.

3. The Regnase-1 inhibitor for use according to claim 1 or 2, wherein the Regnase-1 inhibitor is a nucleic acid capable of inhibiting the expression of Regnase-1.

4. The Regnase-1 inhibitor for use according to claim 1 or 2, wherein the Regnase-1 inhibitor is a gene therapy drug targeting a Regnase-1 gene.

5. The Regnase-1 inhibitor for use according to claim 1 or 2, wherein the Regnase-1 inhibitor is an antibody or peptide capable of specifically binding to Regnase-1.

6. A Regnase-1 inhibitor for use in the improvement of metabolic syndrome.

7. The Regnase-1 inhibitor for use according to claim 6, wherein the Regnase-1 inhibitor is a nucleic acid capable of inhibiting the expression of Regnase-1.

8. The Regnase-1 inhibitor for use according to claim 6, wherein the Regnase-1 inhibitor is a gene therapy drug targeting a Regnase-1 gene.

9. The Regnase-1 inhibitor for use according to claim 6, wherein the Regnase-1 inhibitor is an antibody or peptide capable of specifically binding to Regnase-1.

10. Non-therapeutic use of a Regnase-1 inhibitor for the reduction of body fat.

11. The use according to claim 10, wherein the Regnase-1 inhibitor is a nucleic acid capable of inhibiting the expression of Regnase-1.

12. The use according to claim 10, wherein the Regnase-1 inhibitor is a gene therapy drug targeting a Regnase-1 gene.

13. The use according to claim 10, wherein the Regnase-1 inhibitor is an antibody or peptide capable of specifically binding to Regnase-1.

14. An in vitro method for screening for a substance capable of reducing body fat, the method comprising selecting a substance capable of inhibiting the expression of Regnase-1 or a substance capable of inhibiting the function of Regnase-1.

15. The method according to claim 14, wherein the method comprises
contacting a test substance with cells expressing Regnase-1,
measuring the expression level of Regnase-1 in the cells, and
comparing the expression level of Regnase-1 in the cells in contact with the test substance to that in the absence of the test substance to identify a substance capable of reducing the expression level of Regnase-1.

16. The method according to claim 14, wherein the method comprises
contacting a test substance with Regnase-1 and an RNA substrate,
measuring the degradation level of the RNA substrate, and
comparing the degradation level of the RNA substrate in contact with Regnase-1 in the presence of the test substance to that in the absence of the test substance to identify a substance capable of reducing the degradation level of the RNA substrate.

## Patentansprüche

1. Regnase-1-Inhibitor zur Verwendung bei der Vorbeugung und/oder Behandlung von Fettlebererkrankungen.

2. Regnase-1-Inhibitor zur Verwendung gemäß Anspruch 1, wobei die Fettlebererkrankung eine nichtalkoholische Fettlebererkrankung oder eine nichtalkoholische Steatohepatitis ist.

3. Regnase-1-Inhibitor zur Verwendung gemäß Anspruch 1 oder 2, wobei der Regnase-1-Inhibitor eine Nukleinsäure ist, welche die Expression von Regnase-1 hemmen kann.

4. Regnase-1-Inhibitor zur Verwendung gemäß Anspruch 1 oder 2, wobei der Regnase-1-Inhibitor ein Gentherapeutikum ist, das auf ein Regnase-1-Gen abzielt.

5. Regnase-1-Inhibitor zur Verwendung gemäß Anspruch 1 oder 2, wobei der Regnase-1-Inhibitor ein Antikörper oder Peptid ist, der/das sich gezielt an Regnase-1 binden kann.

6. Regnase-1-Inhibitor zur Verwendung in der Verbesserung des Stoffwechselsyndroms.

7. Regnase-1-Inhibitor zur Verwendung gemäß Anspruch 6, wobei der Regnase-1-Inhibitor eine Nukleinsäure ist, welche die Expression von Regnase-1 hemmen kann.

8. Regnase-1-Inhibitor zur Verwendung gemäß Anspruch 6, wobei der Regnase-1-Inhibitor ein Gentherapeutikum ist, das auf ein Regnase-1-Gen zielt.

9. Regnase-1-Inhibitor zur Verwendung gemäß Anspruch 6, wobei der Regnase-1-Inhibitor ein Antikörper oder Peptid ist, der/das sich gezielt an Regnase-1 binden kann.

10. Nicht-therapeutische Verwendung eines Regnase-1-Inhibitors zur Reduzierung von Körperfett.

11. Verwendung gemäß Anspruch 10, wobei der Regnase-1-Inhibitor eine Nukleinsäure ist, welche die Expression von Regnase-1 hemmen kann.

12. Verwendung gemäß Anspruch 10, wobei der Regnase-1-Inhibitor ein Gentherapeutikum ist, das auf ein Regnase-1-Gen abzielt.

13. Verwendung gemäß Anspruch 10, wobei der Regnase-1-Inhibitor ein Antikörper oder Peptid ist, der/das sich gezielt an Regnase-1 binden kann.

14. In-vitro-Verfahren zum Screening einer Substanz, die Körperfett reduzieren kann, wobei das Verfahren die Auswahl einer Substanz umfasst, die die Expression von Regnase-1 hemmen kann, oder einer Substanz, die die Funktion von Regnase-1 hemmen kann.

15. Verfahren gemäß Anspruch 14, wobei das Verfahren umfasst:
- Inkontaktbringen einer Testsubstanz mit Zellen, die Regnase-1 exprimieren,
- Messen des Expressionsniveaus von Regnase-1 in den Zellen, und
- Vergleichen des Expressionsniveaus von Regnase-1 in den Zellen, die mit der Testsubstanz in Kontakt stehen, mit dem Expressionsniveau in Abwesenheit der Testsubstanz, um eine Substanz zu identifizieren, die in der Lage ist, das Expressionsniveau von Regnase-1 zu reduzieren.

16. Verfahren gemäß Anspruch 14, wobei das Verfahren umfasst
- Inkontaktbringen einer Testsubstanz mit Regnase-1 und einem RNA-Substrat,
- Messen des Abbauniveaus des RNA-Substrats, und
- Vergleichen des Abbauniveaus des RNA-Substrats in Kontakt mit Regnase-1 in Gegenwart der Testsubstanz, mit jenem in Abwesenheit der Testsubstanz, um eine Substanz zu identifizieren, die das Abbauniveau des RNA-Substrats verringern kann.

## Revendications

1. Inhibiteur de la Regnase-1 pour la prévention et/ou le traitement de la stéatose hépatique.

2. Inhibiteur de la Regnase-1 utilisé selon la revendication 1, dans lequel la stéatose hépatique est une stéatose hépatique non alcoolique ou une stéatohépatite non alcoolique.

3. Inhibiteur de la Regnase-1 utilisé selon la revendication 1 ou 2, dans lequel l'inhibiteur de la Regnase-1 est un acide nucléique capable d'inhiber l'expression de la Regnase-1.

4. Inhibiteur de la Regnase-1 utilisé selon la revendication 1 ou 2, dans lequel l'inhibiteur de la Regnase-1 est un médicament de thérapie génique ciblant un gène de la Regnase-1.

5. Inhibiteur de la Regnase-1 utilisé selon la revendication 1 ou 2, dans lequel l'inhibiteur de la Regnase-1 est un anticorps ou un peptide capable de se lier spécifiquement à la Regnase-1.

6. Inhibiteur de la Regnase-1 pour l'amélioration du syndrome métabolique.

7. Inhibiteur de la Regnase-1 utilisé selon la revendication 6, dans lequel l'inhibiteur de la Regnase-1 est un acide nucléique capable d'inhiber l'expression de la Regnase-1.

8. Inhibiteur de la Regnase-1 utilisé selon la revendication 6, dans lequel l'inhibiteur de la Regnase-1 est un médicament de thérapie génique ciblant un gène de la Regnase-1.

9. Inhibiteur de la Regnase-1 utilisé selon la revendication 6, dans lequel l'inhibiteur de la Regnase-1 est un anticorps ou un peptide capable de se lier spécifiquement à la Regnase-1.

10. Utilisation non thérapeutique d'un inhibiteur de la Regnase-1 pour la réduction de la graisse corporelle.

11. Utilisation selon la revendication 10, dans laquelle l'inhibiteur de la Regnase-1 est un acide nucléique capable d'inhiber l'expression de la Regnase-1.

12. Utilisation selon la revendication 10, dans laquelle l'inhibiteur de la Regnase-1 est un médicament de thérapie génique ciblant un gène de la Regnase-1.

13. Utilisation selon la revendication 10, dans laquelle l'inhibiteur de la Regnase-1 est un anticorps ou un peptide capable de se lier spécifiquement à la Regnase-1.

14. Procédé in vitro de criblage d'une substance capable de réduire la graisse corporelle, le procédé comprenant la sélection d'une substance capable d'inhiber l'expression de la Regnase-1 ou d'une substance capable d'inhiber la fonction de la Regnase-1.

15. Procédé selon la revendication 14, dans lequel le procédé comprend les étapes consistant à
mettre en contact une substance d'essai avec des cellules exprimant la Regnase-1, mesurer le niveau d'expression de la Regnase-1 dans les cellules, et
comparer le niveau d'expression de la Regnase-1 dans les cellules en contact avec la substance d'essai à celui en l'absence de la substance d'essai pour identifier une substance capable de réduire le niveau d'expression de la Regnase-1.

16. Procédé selon la revendication 14, dans lequel le procédé comprend les étapes consistant à
mettre en contact une substance d'essai avec la Regnase-1 et un substrat ARN, mesurer le niveau de dégradation du substrat ARN, et
comparer le niveau de dégradation du substrat ARN en contact avec la Regnase-1 en présence de la substance d'essai à celui en absence de la substance d'essai pour identifier une substance capable de réduire le niveau de dégradation du substrat ARN.
